# FASCICULE DE BREVET EUROPEEN

(11) **EP 1 335 202 B1**
(45) Date de publication et mention de la délivrance du brevet: **04.01.2006**
(21) Numéro de dépôt: 03007646.7
(22) Date de dépôt: 10.08.2000
(51) Int. Cl.: G01N 33/50, G01N 33/94, C12Q 1/28

(54) **Procédé D'Identification de Composés Modulateurs des Neuromédiateurs**
Verfahren zum Auffinden von Verbindungen, die als Modulatoren von Neuromediatoren fungieren
Method for screening compounds being modulators of neuromediators

(30) Priorité: 11.08.1999 FR 9910410
(43) Date de publication de la demande: 13.08.2003
(62) Demande divisionnaire de: 00958662.9
(73) Titulaire: CENTRE NATIONAL DE LA RECHERCHE SCIENTIFIQUE (CNRS), 75016 Paris (FR)
(72) Inventeur: Israel, Maurice, 91440 Bures-Sur-Yvette (FR); Lesbats, Bernard, 91220 Plessis Pate (FR)
(74) Mandataire: Warcoin, Jacques

(56) Documents cités:
- METODIEWA D ET AL: "EVIDENCE FOR A PEROXIDATIC OXIDATION OF NOREPINEPHRINE A CATECHOLAMINE BY LACTOPEROXIDASE" BIOCHEMICAL AND BIOPHYSICAL RESEARCH COMMUNICATIONS, vol. 160, no. 3, 1989, pages 1183-1188, XP001149073 ISSN: 0006-291X
- GALZIGNA L ET AL: "A rat brain fraction and different purified peroxidases catalyzing the formation of dopaminochrome from dopamine" BBA - GENERAL SUBJECTS, ELSEVIER SCIENCE PUBLISHERS, NL, vol. 1427, no. 3, 24 mai 1999 (1999-05-24), pages 329-336, XP004276313 ISSN: 0304-4165
- ISRAEL M ET AL: "A chemiluminescent catecholamine assay: Its application for monitoring adrenergic transmitter release" JOURNAL OF NEUROSCIENCE METHODS 1999 NETHERLANDS, vol. 91, no. 1-2, 15 septembre 1999 (1999-09-15), pages 101-107, XP002244783 ISSN: 0165-0270
- ISRAEL ET AL: 'Glutamate and acetylcholine release from cholinergic nerve terminals, a calcium control of the specificity of the release mechanism' NEUROCHEMISTRY INTERNATIONAL vol. 22, no. 1, 1993, pages 53 - 58

## Description

La présente invention concerne des méthodes pour la mise en évidence ou la caractérisation de composés agissant sur la libération de neuromédiateurs. Elle concerne également des préparation de tissu nerveux utiles pour la mise en oeuvre de ces méthodes. Les composés ainsi identifiés peuvent être utilisés comme agents thérapeutiques ou comme *leads* pour le développement d'agents thérapeutiques.

Conscients des problèmes que posent à notre société les atteintes du système nerveux, qu'elles soient neurodégénératives ou liées au tonus mental (dépression, psychose) ou à des traumatismes, les groupes pharmaceutiques et les organismes de recherche académique ont lancé des projets ambitieux ayant pour but de comprendre et de prévenir ces pathologies. A cet effet de nouvelles thérapies, basées sur la greffe de cellules modifiées sécrétrices de médicament, ainsi que la découverte de nouveaux produits assurant une prévention ou un pré traitement sont devenues des objectifs prioritaires pour de nombreux laboratoires. Il faut cependant réaliser que les neurotransmissions centrales ainsi que les processus qui les régulent sont complexes et qu'il est souvent difficile de situer la cible des neurotoxiques endogènes ou exogènes. Il est donc essentiel de disposer de technologies permettant d'explorer rapidement un nombre important de produits agissant sur une série importante de cibles potentielles.

Les méthodes utilisées aujourd'hui par l'industrie pharmaceutique sont longues, lourdes et laborieuses. Ainsi, ces méthodes reposent essentiellement sur l'étude de ce qui se passe après la libération des neuromédiateurs, c'est-à-dire l'étude des événements post-synaptiques.

La présente invention propose de nouvelles méthodes et compositions permettant d'aborder l'étude, la prévention et le traitement des pathologies du système nerveux avec des chances plus grandes de succès. La présente invention repose notamment sur l'étude de la modulation de la neurotransmission par son aspect pré-synaptique. L'invention permet donc d'agir non pas sur les effets ou sur les symptômes; mais directement sur l'origine et les causes des pathologies. La présente invention permet l'étude simultanée de nombreux composés tests sur des compositions de microcubes de matière cérébrale et offre des niveaux de fiabilité et de reproductibilité importants.

La présente invention concerne une méthode pour la préparation de microcubes calibrés de matière cérébrale de mammifère. Ces préparations tissulaires sont utiles pour la mise en oeuvre du procédé ci-dessus, elles sont obtenues *ex vivo* et sont capables de conserver durant toute l'expérience leurs propriétés physiologiques essentielles. Ces préparations de tissu nerveux permettent des dosages rapides, reproductibles et fiables de nombreux produits agissant sur les mécanismes synaptiques. L'invention concerne tout particulièrement des préparations de microcubes de matière cérébrale, qui permettent de tester un grand nombre de produits à partir d'un seul animal, alors que les techniques antérieures nécessitent le sacrifice d'un animal par molécule ou sont moins significatives.

L'invention a pour objet une méthode pour la préparation de microcubes calibrés de matière cérébrale de mammifère comprenant les étapes suivantes :
(i) sélectionner un cerveau entier de mammifère,
(ii) le découper en cubes,
(iii) laver les cubes dans un volume de milieu de Krebs mammifères,
(iv) passer les cubes à travers une ou plusieurs grilles dont les mailles ont un diamètre défini,
(v) récupérer les microcubes calibrés.

Les microcubes présentent l'avantage de conserver les connectivités nerveuses locales et d'être suffisamment oxygénés en milieu physiologique pour assurer une viabilité compatible avec les méthodes de l'invention.

Il convient de rappeler que le fractionnement du système nerveux central a conduit, dans les années 1960, à la purification des terminaisons nerveuses (synaptosomes). Depuis cette époque, l'intérêt des neurobiologistes pour ces préparations ne s'est jamais tari. Si ces préparations ont été très utiles pour déterminer la composition biochimique des éléments synaptiques ou pour isoler les protéines qui assurent la neurotransmission, il faut cependant constater que ces études physiologiques et pharmacologiques se sont heurtées à des difficultés liées à la durée de vie brève *in vitro* de ces synaptosomes, obtenus dans des milieux de fractionnement non adaptés à l'étude physiologique. L'isolement des synaptosomes d'organe électrique de Torpille dans des milieux physiologiques semblait alors être une exception puisqu'ils étaient capables de conserver leurs propriétés physiologiques (potentiel de membrane, synthèse et libération d'ACh) pendant au moins 48 heures. Toutefois, des préparations de tissu nerveux mammifère conservant de telles propriétés physiologiques n'a pas été décrite jusqu'à présent. Les microcubes de matière cérébrale selon l'invention présentent donc un intérêt pharmacologique considérable.

Les microcubes de matière cérébrale constituent une préparation qui offre de multiples avantages. Ainsi, un échantillon de microcubes obtenu à partir d'un cortex est statistiquement comparable à ce cortex entier dans le sens où l'ensemble des neurones contenus dans chaque microcubes se trouve dans un environnement qui est proche de celui qui est le sien dans l'animal entier. Chacun des neurones garde un certain nombre de ses connexions et l'environnement glial reste très stable. Ces deux constances physiologiques sont sans aucun doute responsables de la stabilité et de la longue durée de vie des échantillons de micro cubes. Et: même si tous les microcubes ne sont pas équivalents entre eux du fait de l'hétérogénéité du cortex, chaque population constituant l'échantillon est statistiquement identique.

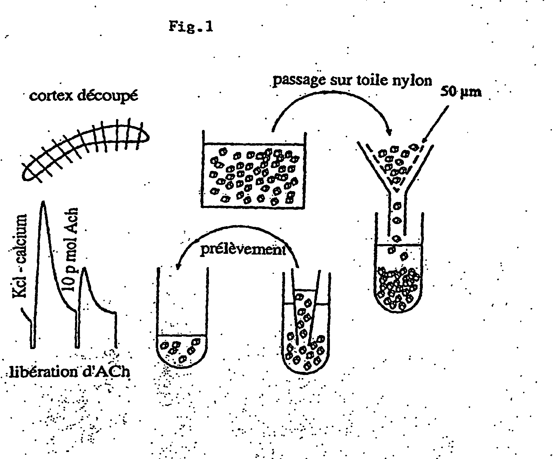

Préférentiellement, les microcubes ont une taille moyenne comprise entre 0,1 et 5 mm³, de préférence entre 0,1 et 3 mm³, encore plus préférentiellement de 1 mm³ environ. Les microcubes sont préparés à partir d'un cerveau entier, par découpage puis passage à travers une où plusieurs grilles de diamètre défini, par exemple entre 0,01 et 2 mm². Il peut s'agir notamment d'une toile de nylon, d'une grille métallique, ou de tout autre support adapté au tamisage. Une illustration de la préparation de microcubes est représentée sur la Figure 1 en annexe, utilisant comme tissu nerveux de départ le cortex cérébral.

Dans un mode particulier de réalisation du procédé de l'invention, après préparation des microcubes et d'un nombre conséquent d'échantillons répartis dans des supports de titration, par exemple des tubes coniques ou des puits de microplaques, on met en contact le ou les composés à tester avec la préparation de cerveau entier, puis l'on prélève le surnageant sur lequel on effectue le dosage (notamment par chimiluminescence) des médiateurs éventuellement libérés par la préparation de cerveau entier.

Un autre avantage des microcubes réside dans leur stabilité importante, même après congélation/décongélation. De ce fait, la mise en batterie de ces dosages n'est pas limitée par la durée de la journée de travail, mais les préparations de cerveau entier peuvent être conservées pour usage ultérieur. Une même préparation permet donc de déterminer avec certitude les effets d'une ou plusieurs séries de composés sur plusieurs (notamment cinq) neurotransmissions en parallèle, ce qui rend les comparaisons d'autant plus faciles que la constitution du cortex des animaux sains est stable.

Ces micro cubes offrent en plus des avantages par rapport aux autres préparations existantes et utilisées par I'industrie pharmaceutique :
- Comparé aux coupes de cerveau, les échantillons de microcubes sont statistiquement plus homogènes car il est toujours difficile de réaliser deux coupes identiques pour avoir une bonne comparaison entre les manipulations,
- Comparé aux synaptosomes, les échantillons de microcubes sont plus stables et ont une durée de vie plus longue car ils ont conservé leur environnement physiologique,
- Comparé aux études sur l'animal vivant, les microcubes permettent de réaliser, à partir d'un seul sacrifice, une batterie de tests concernant à la fois plusieurs composés et plusieurs pans de la neurotransmission, ce qui tranche avec les études comportementales, où un animal testé permet de ne répondre qu'à une question (actif ou pas sur un comportement dont la portée est extrapolée).

L'invention a donc encore pour objet toute composition comprenant des microcubes de matière cérébrale tels qu'obtenus par la susdite méthode. Il s'agit préférentiellement d'une composition comprenant au moins 100 microcubes de matière cérébrale tels que décrits ci avant. L'invention concerne aussi l'utilisation de tels microcubes, ou de toute autre préparation conservant les connectivités locales, pour l'identification de composés modulant la libération de neuromédiateurs.

Une préparation de tissu nerveux peut également être obtenue à partir de fibres moussues de matière cérébrale mammifère, notamment des fibres moussues du cervelet ou de l'hippocampe.

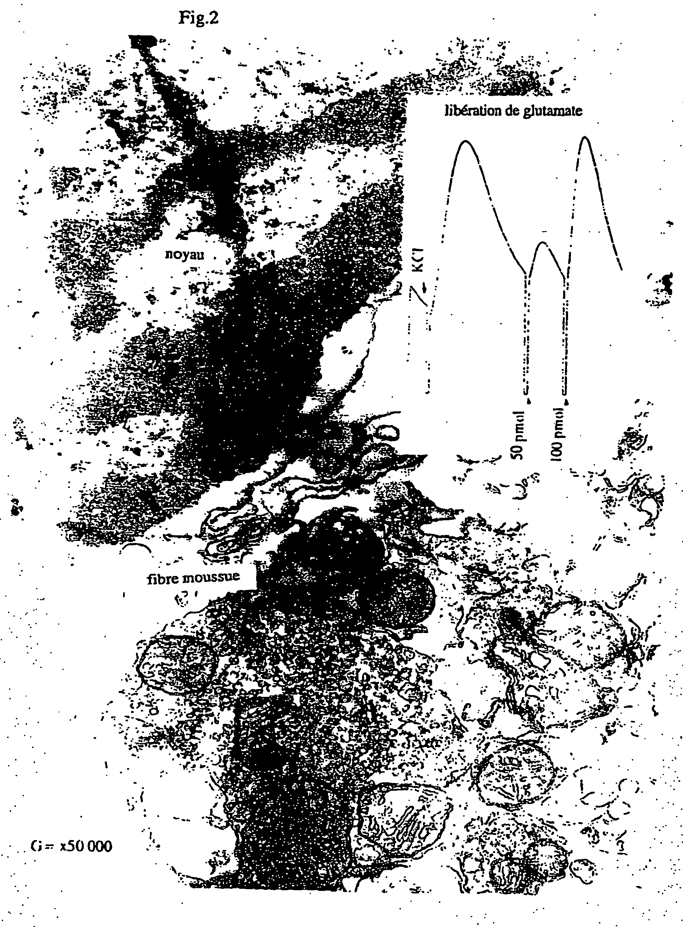

Les fibres moussues contiennent des terminaisons nerveuses glutamatergiques d'un diamètre de 5 a 10 µm. Celles-ci ne sont, en général, pas préservées lorsque l'on homogénéise le tissu nerveux pour isoler des petits synaptosomes (0.5 µm). On utilise comme préparation de tissu nerveux des fibres moussues, préparées de manière à conserver lesdites terminaisons nerveuses. Ces fibres peuvent être préparées par fragmentation du tissu de manière plus douce, dans une solution physiologique, ce qui permet de préserver ces grandes terminaisons glutamatergiques. Des fibres moussues préparées dans ces conditions et utilisables pour la mise en oeuvre de la présente invention sont décrites sur la Figure 2 en annexe.

D'autres préparations de tissu nerveux peuvent être utilisées, telles que des lignées cellulaires exprimant des phénotypes de neurotransmission, des coupes de cerveau, le liquide céphalo-rachidien, etc. Toutefois, il est particulièrement préféré, pour les raisons évoquées ci avant, d'utiliser des préparations conservant les connectivités locales et qui peuvent être produites en nombre important à partir d'un seul animal, telles que des microcubes.

La présente invention concerne également un procédé d'identification de composés capables de moduler la libération de neuromédiateur, caractérisé en ce que l'on met en contact au moins un composé, à tester avec une composition de microcubes calibrés de matière cérébrale de mammifère de l'invention et en ce que l'on détermine l'éventuel effet modulateur dudit composé sur la libération de neuromédiateur par ladite préparation de tissu nerveux.

La présente invention est remarquable en ce qu'elle offre une série de dosages de neurotransmetteurs, rapides, simples, sensibles et fiables, qui peuvent être utilisés en temps réel sur des préparation vivantes, telles que cerveaux perfusés, coupes de tissus, terminaisons nerveuses isolées (synaptosomes), cultures de cellules, etc. Le procédé selon l'invention s'intéresse à la libération des neuromédiateurs, c'est à dire au plan pré-synaptique de la neurotransmission, alors que les techniques d'investigation décrites dans l'art antérieur concernent sur les aspects post-libératoires, comme l'action sur les récepteurs, les inhibiteurs de recapture ou d'enzyme de dégradation des neuromédiateurs.

Selon une forme de réalisation préférée, le procédé de l'invention est caractérisé en ce que l'on met en contact au moins un composé à tester avec une composition de microcubes de l'invention, puis l'on met ladite composition ou son surnageant en présence d'une ou plusieurs substances dont l'une au moins est capable de réagir avec un neuromédiateur un premier temps avec la solution comprenant une ou plusieurs substances dont l'une au moins est capable de réagir avec un neuromédiateur. Ceci permet éventuellement d'éliminer le bruit de fond lié à la présence éventuelle de neuromédiateur résiduel dans le milieu. Dans un second temps, le ou les composés testés sont ajoutés, et la substance détectable est dosée.

Le procédé de l'invention permet, outre de tester plusieurs composés, mais aussi le dosage de plusieurs neuromédiateurs en parallèle. Ainsi, un même composé peut être testé en parallèle pour ses propriétés sur la libération de différents neuromédiateurs, sur des échantillons de tissu nerveux préparés à partir d'un seul animal.

Les résultats obtenus avec le procédé de l'invention et rapportés dans la partie expérimentale ci-après montrent que ce procédé peut être mis en oeuvre de manière aisée, reproductible et rapide, sur des échantillons nombreux et biologiquement significatifs de tissu nerveux, pour mettre en évidence de nouveaux composés ou de nouvelles propriétés de composés connus, ou pour affiner le mécanisme d'action ou la spécificité d'action de composés connus. Les compositions et procédés de l'invention sont ainsi utilisables pour la recherche ou le développement de produits thérapeutiques, ou pour toute autre application dans le domaine du système nerveux.

La mise en contact du ou des composés à tester avec la composition de l'invention peut être réalisée dans tout dispositif approprié à une réaction de dosage, tel que des tubes, puits, plaques, support, etc. Il s'agit préférentiellement de tubes ou de puits de microtitration. Typiquement, les dosages sont réalisés sur des plaques de titration.

La détection ou le dosage d'une ou plusieurs substances détectables si. un neuromédiateur est libéré, ou d'au moins un produit résultant de la transformation de l'une desdites substances si un neuromédiateur est libéré, peut être effectué de différentes façons, selon la nature de la substance à doser.

Dans un mode particulièrement préféré de mise en oeuvre, le procédé utilise des substances froides, c'est-à-dire non radioactives. Le procédé concerne tout particulièrement la détection ou le dosage d'une substance conduisant à une émission de lumière en présence de neuromédiateur, le dosage de la substance consistant alors en le dosage ou la détection de la lumière émise.

Ainsi, dans un mode tout préféré, le procédé de l'invention comprend un dosage par chimiluminescence, ce qui permet des dosages rapides, précis et nombreux des neuromédiateurs. Les substances ou mélanges de produits conduisant à une émission de lumière en présence de neuromédiateur peuvent être par exemple, selon le neuromédiateur, une combinaison de luminol et de péroxydase, de décaldéhyde et de luciférase ou par exemple la NADH-FMN oxydoréductase.

Ainsi, le procédé selon l'invention est caractérisé en ce que l'on en ce que l'on met en contact au moins un composé à tester avec une préparation de tissu nerveux en présence d'une une plusieurs enzymes dont le substrat de l'une au moins est un neuromédiateur et d'au moins un agent capable d'émettre de la lumière suite à la dégradation dudit neuromédiateur par son enzyme.

Comme indiqué précédemment, le dosage du neuromédiateur peut être effectué dans le surnageant de culture des compositions de l'invention, après traitement de celui-ci pour transformer le neuromédiateur en substrat de la réaction d'émission de lumière. Ainsi, dans un mode particulier, le dosage consiste à détecter un produit de transformation du neuromédiateur. Pour cela, le neuromédiateur est préalablement traité pour produire ce produit de transformation. Différents traitements peuvent être utilisés, selon le neuromédiateur concerné.

A titre de neuromédiateur dont la libération est détectée ou dosée selon le procédé de l'invention, on peut citer notamment le glutamate (Glu), l'acétycholine (Ach), le γ-aminobutyrate (Gaba), les catécholamines notamment la dopamine (Da), ou l'ATP.

L'application du procédé de l'invention au dosage ou à la détection de l'acétylcholine est caractérisé en ce que l'on en ce que l'on met en contact au moins un composé à tester avec une préparation de tissu nerveux puis l'on met ladite préparation ou son surnagent en présence, successivement ou simultanément, d'acétylcholinestérase, de cholineoxydase, de peroxydase, et de luminol, et en ce que l'on détecte ou l'on dose la lumière émise par la dégradation du luminol suite à la dégradation par l'acétylcholinestérase de l'acétylcholine libéré par la préparation de tissu nerveux.

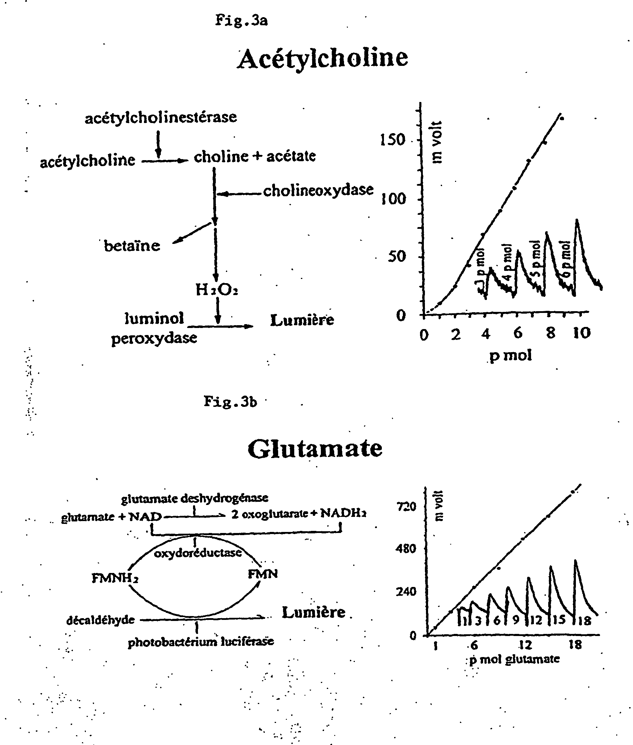

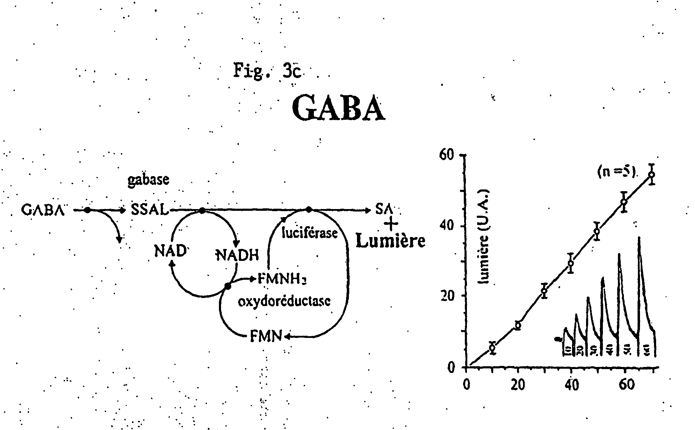

La figure 3a en annexe représente le dosage de l'acétylcholine (Ach). Pour ce dosage le médiateur est hydrolysé par l'acétylcholinestérase, et la choline qui en résulte est oxydée par la cholineoxydase en présence de luminol et de peroxydase. Même lorsqu'un échantillon contient de la choline et de l'ACh, on peut lire séquentiellement les deux substances : lorsque la luminescence due à la choline s'éteint, on ajoute de l'acétylcholinestérase et la lumière qui apparaît donne le taux d'ACh. Ce type de test a été appliqué sur certains échantillons nerveux, essentiellement non mammifère ou ne permettant pas des études sur des gammes assez larges de composés (Israel et Morel, Rev. Neurol. 143 (1987) 89). La présente invention montre à présent que ce type de test peut être utilisé à grande échelle, sur des préparation particulières de tissu nerveux, et en batterie avec d'autres tests de dosage de neuromédiateur.

L'application du procédé de l'invention au dosage ou à la détection du plutamate est caractérisé en ce que l'on met en contact au moins un composé à tester avec une préparation de tissu nerveux, puis l'on met ladite préparation ou son surnageant en présence, successivement ou simultanément, de glutamate desydrogénase, d'oxydoréductase, de luciférase, de NAD et de décaldéhyde, et en ce que l'on détecte ou l'on dose la lumière émise par la dégradation du décaldéhyde suite à la dégradation par la glutamate desydrogénase du glutamate libéré par la préparation de tissu nerveux.

La figure 3b en annexe représente le dosage du glutamate. Pour ce dosage le glutamate est par exemple déshydrogéné par l'enzyme glutamate deshydrogénase ; le NADH₂ qui apparaît étant ensuite attaqué par l'enzyme NADH-FMN oxydoréductase, ce qui déclenche en présence de décaldéhyde et de luciférase de photobactérium, une émission lumineuse. Ce type de dosage a été mis en oeuvre dans d'autres conditions, expérimentales, sur des terminaisons nerveuses d'organes électriques (Israel et al., Neurochem. Int. 22 (1993) 53). La présente invention montre à présent que ce type de test peut être utilisé à grande échelle, sur des préparation particulières de tissu nerveux, et en batterie avec d'autres tests de dosage de neuromédiateur.

L'application du procédé de l'invention au dosage ou à la détection du Gaba est caractérisé en ce que l'on met en contact au moins un composé à tester avec une préparation de tissu nerveux puis l'on met ladite préparation ou son surnageant en présence, successivement ou simultanément, de gabase, d'oxydoréductase, de luciférase, de NAD et de FMN et en ce que l'on détecte ou l'on dose la lumière émise par la dégradation du SSAL suite à la dégradation par la gabase du Gaba libéré par la préparation de tissu nerveux.

La figure 3c en annexe représente le dosage du Gaba. Pour ce dosage, l'enzyme Gabase est utilisée pour convertir le Gaba en aldéhyde semi succinique avec production de NADH₂. L'aldéhyde semi succinique et le NADH déclenchent, en présence de NADH-FMN oxydoréductase, l'émission de lumière. L'avantage de ce test est qu'il n'est pas nécessaire d'ajouter un aldéhyde exogène. Ce type de dosage a été mis en oeuvre dans d'autres conditions, expérimentales, sur des terminaisons nerveuses d'organes électriques (Israel et Lesbats., J. Neurochem. 67 (1996) 2624). La présente invention montre à présent que ce type de test peut être utilisé à grande échelle, sur des préparations particulières de tissu nerveux, et en batterie avec d'autres tests de dosage de neuromédiateur.

Pour le dosage ou la détection des catécholamines, l'on met en contact au moins un composé à tester avec une préparation de tissu nerveux puis l'on met ladite préparation ou son surnagent en présence, successivement ou simultanément, de lactopéroxydase, d'O₂ et de luminol et l'on détecte ou l'on dose la lumière émise par la dégradation du luminol suite à la dégradation par la lactopéroxydase d'une catécholamine libérée par la préparation de tissu nerveux.

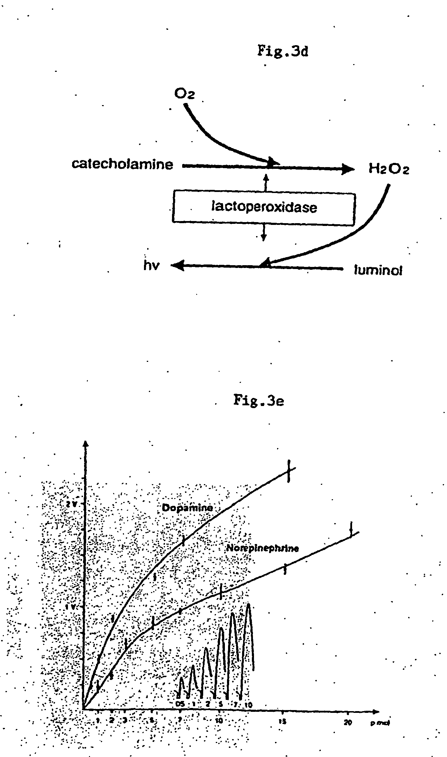

Les figures 3d et 3e en annexe représentent le dosage des catécholamines, notamment de la dopamine. Pour le dosage de la dopamine, un test particulièrement efficace est basé sur la mise en évidence de propriétés d'une enzyme dans la transformation de la dopamine. Ainsi, ce test montre que l'enzyme lactopéroxydase, en présence de O₂, conduit à la formation de H₂O₂, puis, en présence de ce dernier et de luminol, la même lactopéroxydase catalyse la formation de lumière. De ce fait, de manière inattendue, la lactopéroxydase apparaît comme possédant à la fois une action péroxydase classiquement décrite et une action oxydase.

Un autre test basé sur une catécholamine luminescente et ses applications permet de contrôler le relargage d'un transmetteur adrénergique. Un nombre croissant d'oxidases a été utilisé pour mesurer leur substrat spécifique (glucose, xanthine, choline, etc (Michelson 1978 et Takayama et al. 1977). Dans le cas du transmetteur acétylcholine (Ach) il a été proposé en 1980 un procédé dans lequel la choline résultant de l'hydrolyse de l'ACh était oxydée par la choline oxydase en formant du péroxyde d'hydrogène, ce dernier étant détecté en utilisant une réaction chimiluminescente (luminol plus péroxydase) (Israel et Lesbats, 1981 a, b, 1982, 1987). Pour d'autres transmetteurs tels le glutamate (Glu) ou le γ aminobutyrate (Gaba), le test chimiluminescent correspondant a été trouvé, Glu et Gaba sont des substrats pour des deshydrogénases donnant du NADH qui déclenche la luminescence des luciférases bactériennes sensibles aux aldéhydes (Fosse et al, 1986 **;** Israel et al, 1993 ; Israel et Lebats, 1996)**.** Si l'on ajoute à ces transmetteurs l'ATP, détecté par les luciférases de luciole (De Luca et Mc Elroy, 1978 ; Meunier et al, 1975) on se rend compte que beaucoup de transmetteurs sont maintenant mesurés à l'aide de méthodes chimiluminescentes. Dans le cas des monoamines, la procédure de Tenne pour la monoamine oxydase convertit les amines aliphatiques en aldéhydes aliphatiques qui déclenchent la luminescence de systèmes bactériens sensibles aux aldéhydes à longue chaîne (Tenne et al, 1985 a, b).

L'action de la. monoamine oxydase sur les catécholamines produit du péroxyde d'hydrogène (Cadet et Brannock, 1998) qui a pu être détecté à l'aide du luminol. Il a maintenant été mis en évidence dans le cadre de la présente invention que la lactopéroxydase de lait présente une fonction mixte oxydase - péroxydase, elle a été capable d'oxyder des catécholamines et de catalyser la réaction du peroxyde formé avec le luminol, générant ainsi l'émission de lumière. Ainsi, il a été mis au point un test sensible qui mesure aussi bien la norépinephrine, l'épinephrine ou la dopamine, il réagit très faiblement avec la L-DOPA ou. la sérotonine. Ce test a été appliqué pour contrôler le relargage de catécholamines des tissus et des cellules adrénergiques.

Le procédé résultant permet le dosage de catécholamine dans un échantillon, comprenant la mise en contact de cet échantillon avec la lactopéroxydase et le luminol, et le dosage de la lumière émise. L'échantillon peut être tout surnageant de culture de cellules nerveuses, de microcubes de cerveau, ou de toute autre préparation de tissu nerveux, éventuellement. après stimulation de la libération de catécholamines par dépolarisation au calcium et incubation en présence du composé test. Ce dosage peut être utilisé pour doser l'épinéphrine, la norépinéphrine, la dopamine, etc.

Pour la mise en **oe**uvre d'un procédé décrit précédemment un kit peut comprendre:
- une ou plusieurs préparations de tissus nerveux dans des compartiments séparés,
- une ou plusieurs substances dont l'une au moins est capables de réagir avec un neuromédiateur libéré par la préparation de tissu nerveux, et l'une au moins desdites substances est détectable si un neuromédiateur est libéré ou dont au moins un produit résultant de la transformation de l'une desdites substances est détectable si un neuromédiateur est libéré.
- éventuellement un ou plusieurs composés de référence dont l'effet sur la libération d'un ou plusieurs neuromédiateurs par la préparation de tissu nerveux est connu.

La mise en contact du ou des composés à tester avec la préparation de tissu nerveux, dans un tel kit, peut être réalisée dans tout dispositif approprié à une réaction de dosage, tel que des tubes, puits, plaques, support, etc. Il s'agit préférentiellement de tubes ou de puits de microtitration. Typiquement, les dosages sont réalisés sur des plaques de titration.

Les composés capables de moduler la libération pré synaptique de neuromédiateurs identifiable par un procédé selon l'invention, peuvent être utilisés pour la fabrication d'un médicament destiné au traitement ou à la prévention de maladies du système nerveux.

En effet, les composés identifiés ou caractérisés selon le procédé de l'invention peuvent soit augmenter, soit inhiber la libération de médiateur. Ainsi, selon les pathologies concernées, il peut être avantageux de chercher à augmenter ou à réduire la quantité de médiateur libérée. A cet égard, les composés identifiés peuvent constituer des composés à usage thérapeutique, ou bien des leads (cibles) pour le développement de tels agents thérapeutiques. Les applications de ces composés sont par exemple :
- pour les composés modulant la libération d'acétylcholine, la neurodégénérecence et la maladie d'Alzheimer,
- pour les composés modulant la libération de glutamate, la neurotoxicité et l'ischémie,
- pour les composés modulant la libération de Gaba, l'épilepsie et l'anxiété,
- pour les composés modulant la libération de catécholamines (dopamine), la psychose, la dépression, la maladie de Parkinson,
- pour les composés modulant la libération d'ATP, l'anxiété, la panique, les phobies, la maladie de parkinson.

D'autres avantages et caractéristiques de l'invention apparaîtront des exemples qui suivent, donnés à titre illustratif et non limitatif, et qui décrivent les dosages en chimiluminescence de quatre médiateurs (ACh, Glu, Gaba et Da) sur une préparation de microcubes de matière cérébrale et sur des fibres moussues. Bien entendu d'autres préparations, entrant dans le cadre de la présente invention sont également disponibles, telles que les cellules exprimant divers phénotypes de neurotransmission, la mesure du glutamate du liquide céphalo-rachidien pour évaluer l'excito-toxicité.

Il sera fait référence dans ce qui suit aux dessins en annexe, dans lesquels :
- La Figure 1 représente la production de microcubes de matière cérébrale, et leur utilisation pour le dosage de neuromédiateurs.
- La Figure 2 représente la production et l'aspect de fibres moussues glutamatergiques, et leur utilisation pour le dosage de neuromédiateurs.
- La Figure 3 représente le principe des dosages de l'acétylcholine (Figure 3a), du glutamate (Figure 3b) et du Gaba (Figure 3c). La Figure 3d représente le principe du test catécholamine-luminescent, la Figure 3e représente le dosage de la dopamine et de la norépinéphrine. Les différents dosages permettent de détecter quelques picomoles de l'un des médiateurs. Une proportionnalité entre la lumière émise et le taux de médiateur est montrée sur les figures pour chaque médiateur.
- La Figure 4 représente le remplissage de cellules en neuromédiateurs et leur utilisation pour le dosage de neuromédiateurs. Les cellules sont chargées en neuromédiateur en l'ajoutant au milieu de culture (10 mM). Les cellules sont lavées 5 à 6 fois, puis reprises dans un volume de milieu, pour aboutir à une concentration de 1 nanomole de neuromédiateur dans 5 µl environ. Différents phénotypes de neurotransmetteurs sont ainsi exprimés par les lignées cellulaires.
- La Figure 5 représente le dosage du glutamate dans le liquide céphalo-rachidien.
- La Figure 6 représente le dosage des catécholamines sur une préparation de cellules adrénergiques dissociées, en solution saline (A) ou non saline (B).
- La Figure 7 représente le dosage des catécholamines sur une préparation de microcubes de cerveau stimulée par du KCl en présence de calcium (7A) ou d'EGTA (7B).
- La Figure 8 représente la détermination du contenu en catécholamines des extraits biologiques.
- La Figure 9 représente le relargage de catécholamine par des fragments de glande surrénale.
- La Figure 10 représente le relargage de catécholamines par des cellules de neuroblastome enrichies en dopamine au cours de leur culture.

### Exemple 1 : Matériel et Méthodes.

La lactopéroxydase (EC1.11.1.7) de lait bovin a été obtenue de Sigma sous forme de poudre lyophilisée (80-150 Unités / mg de protéine). Le luminol (5-amino-1,2,3,4-tétrahydrophthalazindione-1,4) a été obtenu de Merck. Une solution stock à 1 mM a été préparée en diluant 18 mg dans quelques gouttes de NaOH IM, et le volume final a été porté à 100 mM par ajout de tampon Tris 0,2 M, pH 8,6.

Le milieu de Krebs mammifères est composé de 136 mM NaCl, 5,6 mM KCl, 2,2 mM CaCl2, 1,2 mM MgCl2, 1,2 mM phosphate de sodium, 2,5 mM de bicarbonate de sodium, 5,5 mM glucose, oxygéné

### Exemple 2: Préparation de microcubes de matière cérébrale et utilisations pour le dosage de neuromédiateurs (Figure 1).

Le cortex d'un rat ou d'une souris (ou d'un autre mammifère, y compris humain) est disséqué et découpé en cubes de 1 a 2 mm³ environ avec une lame de scalpel. Ces cubes sont lavés dans un volume de Krebs mammifères (au moins deux fois 250 mL) puis aspirés à l'aide d'une pipette P5000 et forcés au "jet " à travers une toile Nylon rigide ayant une maille de 1 mm de coté environ. Les cubes ainsi calibrés sont récoltés au fond d'un bêcher par sédimentation spontanée ce qui réduit les centrifugations plus "traumatisantes", puis les microcubes sont récoltés dans un volume progressivement plus réduit. Les microcubes sédimentent au fond d'un tube conique et sont couverts par 1 ou 2 mL de Krebs. Lorsque l'on pipette dans un culot de 20 à 50 µL de ces microcubes à l'aide d'une pipette Gilson pointe jaune à l'extrémité coupée pour élargir l'entrée, on prélève un nombre égal de microcubes de sorte que l'on peut tester la libération de médiateur sur des échantillons statistiquement comparables. On peut par exemple incuber des échantillons de 50 µL de microcubes avec une série de composés à différentes concentrations, et analyser leurs effets sur la libération spontanée ou induite par dépolarisation au potassium par rapport à un témoin. Les dosages de quatre neuromédiateurs peuvent être d'autre part répartis sur plusieurs jours puisque ces préparations sont congelables.

Cette méthode a été mise en oeuvre de manière particulièrement efficace dans le cas de l'ACh, du Gaba, du glutamate ou de la dopamine (Figures 3). Pour ce qui est du glutamate, il est ailleurs décrit une préparation plus idoine, consistant en la mesure de la libération de ce médiateur à partir de terminaisons nerveuses de fibres moussues de cervelet ou de l'hippocampe.

### Exemple 3: Préparation de fibres moussues qlutamatergiques du cervelet ou de l'hippocampe et utilisations pour le dosage de neuromédiateurs (Figure 2).

Les terminaisons nerveuses des fibres moussues ont un diamètre de 5 a 10 µm (Fig. 2) et ne sont, en général, pas préservées lorsque l'on homogénéise le tissu nerveux pour isoler des petits synaptosomes (0.5 µm) qui sont habituellement purifiés. Si l'on prend la précaution de fragmenter le tissu de manière plus douce dans une solution physiologique, il est possible de préserver ces grandes terminaisons glutamatergiques. A cet effet, des quantités de tissus de 0.1 g environ ont été dissociées par aspiration-expiration dans un volume de 0.3 mL à l'aide d'une pipette Gilson pointe bleue. Cet "homogénat " est ensuite ramené à 2 mL de Krebs et filtré sur une toile de Nylon de 50 µm. Les terminaisons nerveuses des fibres moussues sédimentent spontanément dans la fraction nucléaire. Ce culot est lavé 1 ou 2 fois dans du Krebs par sédimentation spontanée puis resuspendu dans 1 mL de Krebs. Ces terminaisons nerveuses vont libérer le médiateur lorsqu'on les dépolarise en présence de calcium et seront stables longtemps.

Pour étudier l'effet de composés sur la libération de glutamate, on utilise en général 3 à 5 µl de cette suspension, qui sont ajoutés au milieu réactionnel de dosage du glutamate, la libération étant déclenchée par l'addition de KCl en présence de calcium. Les résultats obtenus montrent que cette préparation de tissu nerveux constitue un matériel de choix pour des dosages répétés, sur un grand nombre d'échantillons.

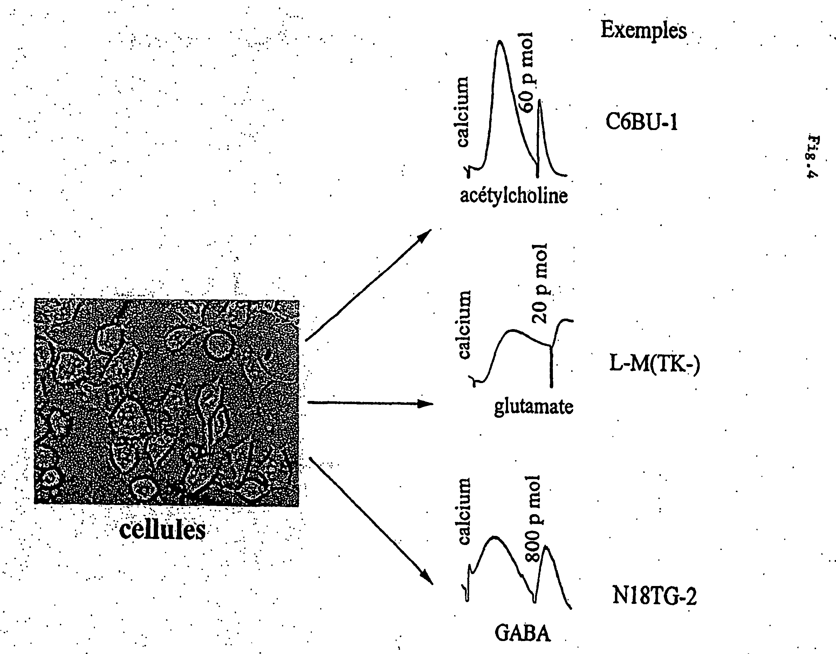

### Exemple 4: Lignées cellulaires exprimant divers phénotypes de neurotransmission (Figure 4).

Il a été montré que l'on pouvait charger des cellules en cultures avec l'un des trois médiateurs ACh, Glu ou Gaba en l'ajoutant durant 5 à 12 heures au milieu de culture (Israël et al., Neuropharmacology 36 (1997) 1789). Les modalités techniques sont différentes pour chaque médiateur. Dans le cas de l'ACh par exemple (Figure 4), il est nécessaire de bloquer au préalable les cholinestérases (phospholine 50 µM). Dans tous les cas, cependant, la concentration intracellulaire de médiateur devient proche de la concentration extracellulaire utilisée (ici 10 mM) mais on peut la porter à 40 mM. Après lavage du milieu, les cellules gardent le médiateur incorporé. La comparaison des contenus en ACh, Gaba et Glu de diverses lignées cellulaires est effectuée en lysant quelques µl de la suspension dans le milieu de mesure, avec 0.5% ou 0.05% triton pour les cellules chargées en ACh ou Glu respectivement, et en les portant préalablement à 80°C pour le Gaba puis en dosant le médiateur ainsi libéré. Certaines lignées cellulaires libèrent préférentiellement l'ACh, d'autres le Gaba ou le glutamate (Fig.4). Même si les microcubes constituent un matériel de choix, ces lignées sont également utilisables dans les procédés de l'invention pour l'identification ou la caractérisation de composés capables de moduler la libération de neuromédiateurs.

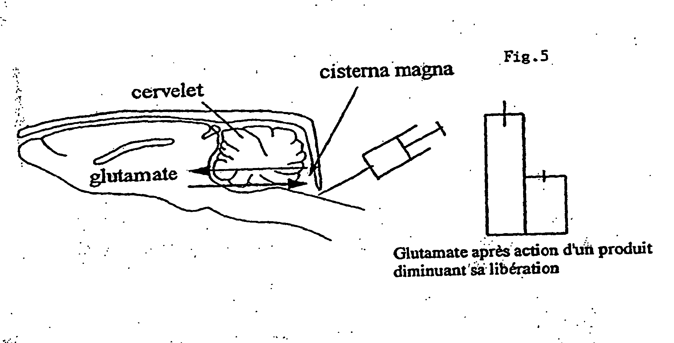

### Exemple 5: Etude de l'excito-toxicité de glutamate mesuré dans le liquide céphalo-rachidien (Figure 5).

Les altérations du système nerveux central consécutives à une lésion locale touchent souvent des territoires tissulaires plus étendus que ceux de la lésion proprement dite. L'aire de diffusion des aminoacides excitateurs (glutamate) libérés autour du site semble correspondre à cet effet excito-toxique. Dans la mesure où l'espace extracellulaire est en équilibre avec le liquide céphalo-rachidien, on peut envisager que des composés qui réduiraient la teneur en glutamate du liquide céphalo-rachidien pourraient limiter l'excito-toxicité et prévenir l'extension des zones d'altération. L'expérience réalisée sur la figure 5 montre, à titre d'exemple, qu'un produit qui diminue la libération de glutamate a entraîné une baisse de glutamate dans le liquide céphalo-rachidien des animaux traités.

### Exemple 6 : Etude de la libération de catécholamines.

L'invention se rapporte tout particulièrement à la mise au point d'un test particulièrement efficace de dosage, par chimiluminescence, des catécholamines dans des préparations de tissu nerveux. Ce test repose sur la double transformation inattendue de catécholamines par la lactoperoxydase, conduisant à l'émission de lumière, comme il est représenté sur la Figure 3d. Pour la mise en oeuvre de ce test, l'échantillon comprenant le neuromédiateur est mis en contact avec la lactopéroxydase et le luminol. De préférence, des volumes égaux de ces réactifs sont préalablement mélangés (par exemple 30 µl chacun), et le mélange est ensuite ajouté à l'échantillon (environ 6 µl). La réaction est préférentiellement réalisée dans une solution saline composée de 136 mM NaCl, 5, 6 mM KCl, 1,2 mM MgCl2. 20 mM tampon Tris pH 8,6, oxygéné (solution I). La solution est placée dans un tube et introduite dans le luminomètre, sous agitation douce. Lorsque l'émission initiale diminue pour atteindre un niveau de base, le mélange peut être utilisé pour doser les catécholamines. Pour un dosage en conditions standard, environ 3 µl de chaque réactif sont utilisés (soit 6 µl du mélange). Pour un dosage moins sensible, on peut utiliser environ 3 µl de luminol et 5 µl de lactopéroxydase dans une solution non saline composée de 120 mM sucrose, 120 mM tampon Tris pH 8, 6, oxygénée (solution II) .

Le dosage de catécholamines a été effectué à partir de différentes préparations de tissu nerveux, incluant les cellules adrénergiques dissociées, les microcubes de régions striatales du cerveau ou des lignées cellulaires chargées en catécholamine.

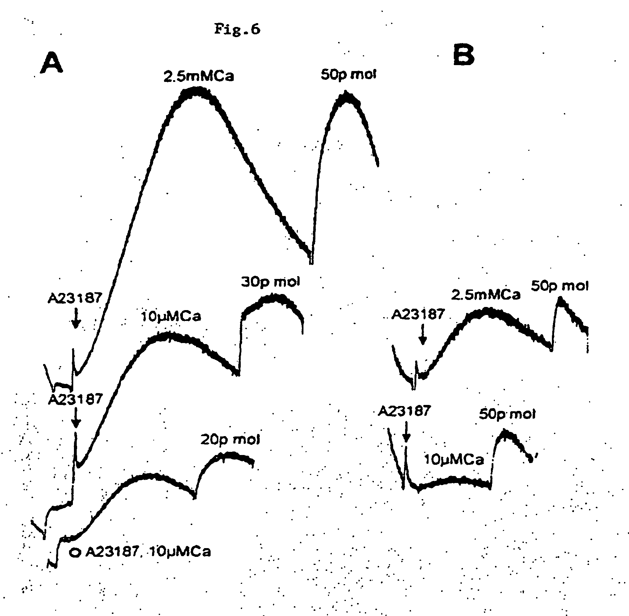

Des cellules adrénergiques dissociées ont été préparées et utilisées dans un test de libération de dopamine. Pour cela, 4 glandes adrénergiques ont été dissociées dans un volume final de 2 ml de milieu Krebs mammifère. La libération a été testée sur des volumes de 5 à 10 µl, dans les solutions I ou II ci-dessus dans lesquelles le luminol (3 µl) et la lactopéroxydase (5 µl) ont été ajoutés. La libération de catécholamine a été stimulée par l'ajout du ionophore calcique A23187 (1,2 µl) en présence de calcium (2.5 mM). Les résultats obtenus sont présentés sur la Figure 6 et montrent que le test de l'invention permet un dosage rapide et sensible de la libération de catécholamine sur de petits échantillons de tissu nerveux.

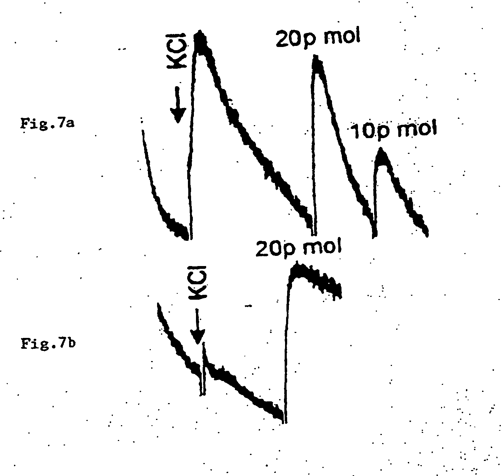

Des microcubes de régions striatales de cerveau ont été préparés à partir du cerveau de deux souris, qui ont été découpés et passés à travers une membrane de nylon (0,5 man de section), puis repris dans un volume final de 2 ml dans un tube conique. Des échantillons de 10 µl du culot ont été utilisés pour effectuer le test de libération, dans les conditions décrites ci avant (dans 2 ml de solution I contenant 3 µl de luminol et 3 µl de lactopéroxydase). La libération a été stimulée par dépolarisation au KC1 (45 mM) en présence de calcium (2,5 mM). Des contrôles sans calcium ont été effectués, en présence de EGTA (7 µM). Les résultats obtenus sont présentés sur la Figure 7, et montrent que le procédé de l'invention est sensible et permet de doser le neurotransmetteur libéré en réponse au calcium. Ce test peut être aisément utilisé pour l'identification ou la caractérisation de composés agissant sur cette libération de dopamine.

Des expériences similaires ont été réalisées à partir de lignées cellulaires chargées en dopamine (notamment de lignées de neuroblastes) et ont permis de confirmer la sensibilité et la reproductibilité du procédé de l'invention.

### Exemple 7 : Etude de la libération de catécholamines.

Cet exemple plus détaillé que l'exemple 5 décrit un test basé sur une catécholamine luminescente et ses applications pour contrôler le relargage d'un transmetteur adrénergique.

### 1) Matériel et méthodes.

### a) Solutions et tampons.

Solution I : consiste en NaCl 136 mM, KCl 5,6 mM, MgCl2 1,2 mM, tampon Tris 20 mM pH 8,6 oxygéné.
solution II : consiste en sucrose 120 mM, tampon Tris 120 mM pH 8,6 oxygéné.
Milieu de Krebs mammifères: NaCl 136 mM, KCl 5,6 mM, CaCl2 2,2 mM, MgC12 1,2 mM, phosphate de Na 1,2 mM, bicarbonate de Na 2,5 mM, glucose 5,5 mM pH 7,4 oxygéné.

### b) Solutions stocks.

La lactopéroxydase (E C 1. 11. 1. 7) de lait de vache a été achetée chez Sigma sous forme d'une poudre lyophilisée (80 à 150 unités par mg de protéine), elle a été dissoute dans 1 ml d'eau et gardée congelée par aliquotes de 100 µl.

Le luminol (5-amino-1,2,3,4-tetrahydrophtala zindione-1,4) a été acheté chez Merck. Une solution stock a été préparée en dissolvant 18 mg de luminol dans quelques gouttes de NaOH 1M et le volume a été porté à 100 ml avec du tampon Tris pH 8,6 0,2 M. Des aliquotes de 1 ml ont été gardées à -20°C.

### c) Mélange réactionnel.

Dans 2 ml de solution I les inventeurs ont ajouté 3 µl de luminol et 3 µl de lactopéroxydase. Il a paru préférable de mélanger un volume égal (30 µl de chaque) et d'ajouter 6 µl du mélange gardé dans la glace. Le tube est placé dans le luminomètre et la solution mélangée avec un petit aimant. Quand l'éclair initial a décliné en une ligne de base qui diminue lentement, le mélange peut être utilisé pour mesurer les catécholamines (norépinéphrine, épinéphrine, dopamine) . Si un test moins sensible est nécessaire, la solution II est utilisée, à laquelle sont ajoutés 3 µl de luminol et 5 µl de lactopéroxydase.

### d) Extraits tissulaires.

50 à 100 mg de tissus sont coupés et extraits pendant deux heures dans 1 ml d'acide trichloroacétique (TCA) refroidi dans la glace, les protéines dénaturées sont centrifugées et le surnageant prélevé. Une aliquote de 0,25 ml a été diluée de moitié dans de l'eau et lavée à l'éther 4 fois pour retirer le TCA (pH final : 4). Les traces d'éther dans la phase aqueuse ont été évaporées. Un échantillon est dilué 1/10 à 1/100 dans la solution I et 0,5 ml sont lavés dans 2 ml de chloroforme. Puis des aliquotes de 40 µl de la phase aqueuse ont été traitées avec 10 µl de Triton X100 à 1% et la détermination a été réalisée, sur 3 à 5 µl**.** Chaque échantillon est comparé aux standards, et il est essentiel de tester un blanc traité exactement comme l'échantillon. Si le niveau du blanc est élevé, il faut diluer le Triton jusqu'à ce que le blanc devienne négligeable par comparaison avec l'échantillon (voir Fig.3).

### e) Relargage de catécholamines de morceaux de surrénales.

Deux souris ont été anesthésiées à l'éther, puis leurs surrénales ont été retirées et coupées 4 en 5 morceaux qui ont été lavés 40 mn et 1 heure dans 400 ml de Krebs mammifère. Le relargage a.été réalisé en solution I ou II dans laquelle le luminol et la lactopéroxydase sont augmentées respectivement jusqu'à 5 et 10 µl. La dépolarisation avec du KCI 60 mM en présence de calcium 2,5 mM déclenche le relargage,

### f) Relargage de catécholamines de cellules adrénergiques dissociées.

Les morceaux ont été lavés comme précédemment et dissociés par plusieurs aspirations et rejets à travers une pipette (Eppendorf, cône de 1 ml) dans un volume final de 2 ml. Le relargage a été testé sur des aliquotes de 5 et 10 µl. Davantage de matériel éteint le mélange réactionnel. Le relargage a été mesuré en solution I ou II dans lesquelles le luminol (3 µl) et la lactopéroxydase (5 µl) ont été ajoutés. Le relargage a été déclenché par l'addition de calcium ionophore A23187 (1,2 µM) en présence de calcium 2,5 mM.

### g) Relargage de catécholamines de micro fragments de cerveau striatal.

Les régions striatales du cerveau de deux souris ont été découpées et passées de force avec de grands volumes de Krebs à travers une gaze rigide de nylon (0, 5 mm). Les micro fragments ont été collectés au fond du bécher et concentrés en un volume final de 2 ml dans un tube conique. Le culot se forme rapidement par sédimentation et il est possible de prélever des échantillons de 10 µl du culot. Ils sont ajoutés dans 2 ml de solution I contenant 6 µl du mélange lactopéroxidase-luminol (un volume égal de chaque solution stock). Le relargage a été obtenu par dépolarisation. au KCl (45 mM) en présence de 2,5 mM de calcium. Les contrôles en absence de calcium ont été réalisés avec 0,7 µM d'EGTA dans la solution. Plus d'EGTA pourrait inhiber la réaction, cette condition donne autant de sensibilité pour les contrôles et pour les échantillons

### h) Relargage de catécholamines de cellules enrichies en dopamine.

Des cultures cellulaires NG108-15 ont été maintenues dans du milieu Eagle modifié du Dulbecco (DMEM) enrichi en glutamine 2 mM, sérum de veau foetal 10%, HAT (hypoxanthine 100 µM, aminopterine 1 mM et thymidine 16 µM). Les cellules ont été cultivées à 37°C dans une atmosphère humidifiée de 95 % d'air et 5% de CO2. Afin de charger les cellules en dopamine, les inventeurs ont ajouté, 16 heures avant l'expérience, un mélange de dopamine et d'acide ascorbique neutralisé pour obtenir une concentration finale de 10 mM de chaque dans le récipient de culture. La solution stock a été faite avec de l'acide ascorbique 0,5 M neutralisé par de la soude et avec de la dopamine 0,5 M pH 8.7. Les cellules sont lavées dans une solution consistant en NaCl 136 mM, KCl S, 6 mM. MgCl2 1,2 mM, tampon Tris 20 mM pH 8,6, glucose 5,5 mM oxygéné. Quatre lavages à 1200 RPM pendant 5 mn ont été réalisés. Le culot provenant des 4 récipients a été resuspendu dans 0,5 ml de Krebs.

Le relargage des cellules enrichies a été réalisé dans 2 ml de solution II contenant 3 µl de luminol et 5 µl de lactopéroxydase, il a été trouvé préférable de renforcer le. mélange réactionnel après avoir ajouté les cellules (5 µl) en doublant le luminol et la péroxydase et en ajoutant 1 ml de plus de solution II. Quand le tracé est revenu à un niveau de base acceptable, le relargage a été déclenché par le calcium (1,6 mM) en présence de l'ionophore A 23187 ( 0 . 7 µM).

### i) Localisation histochimique de la dopamine.

Les cellules ont été diluées de moitié avec une solution à 2 % de volume à volume d'acide glyoxyligue contenant 0,5 mg % MgC12 pH 5. Les cellules ont été déposées sur une lamelle de verre, et la solution drainée. Les cellules ont été séchées à température ambiante puis traitées à 80°C pendant une demi-heure, avant d'être examinées dans le milieu adéquat pour les observations de la fluorescence. La procédure a été adaptée de Liu. et al., 1992 suivant De La Torre 1980 et Knigge et al., 1977.

### j) Fiaures.

La Figure 3d représente le principe du test catécholamine-luminescent.

La lactopéroxydase catalyse l'oxydation des catécholamines (dopamine, norepinephrine, épinephrine) et l'oxydation chimiluminescente du luminol, elle présente une fonction mixte oxydase-péroxydase.

La Figure 3e représente la courbe dose-réponse pour la dopamine et la norépinephrine.

Le mélange réactionnel, la solution I contenant la lactopéroxydase et le luminol(voir méthodes), donne une réaction chimiluminescente quand la catécholamine est ajoutée. Au-dessous des courbes est montrée une réponse typique dans la gamme des picomoles. La différence entre les deux courbes dépend de lot de lactopéroxydase et des solutions, elle ne correspond pas à une distinction spécifique entre les deux catécholamines.

La Figure 6 représente le relargage de catécholamines par des cellules adrénergiques dissociées.

En A, le relargage a été mesuré en solution I saline, il a été induit par l'addition d'ionophore A23107 (1,2 µM) en présence de 2,5 mM de calcium (tracé du haut). Quand le calcium a été réduit jusqu'à 10 µM, le relargage est clairement plus faible mais pas négligeable (tracé du milieu). Sur le tracé du milieu, les pertes spontanées des cellules sont mesurées en absence d'ionophore.

En B, le relargage a été mesuré en solution II (sucrose-Tris) et a été provoqué par l'addition d'ionophore comme en A, en présence de calcium 2,5 mM ou de calcium 10 µM (tracé du bas). Dans cette solution, le relargage apparaît comme négligeable en présence de faibles quantités de calcium, et les pertes sportanées ne son pas mesurables.

La proportion de lactopéroxydase et de luminol est donnée dans la partie Méthodes.

La Figure 7 représente le relargage de catécholamine par des microfragments striataux.

Les morceaux de striatum ont été passés de force, avec un grand volume de Krebs, à travers une gaze de nylon rigide, les microfragments ont ensuite pu sédimenter et le striatum de 4 souris a été concentré dans un volume de 2 ml. Sur le tracé du haut, le relargage de catécholamine (probablement de la dopamine) a été provoqué par le KCl (45 mM) et le calcium (2,5 mM). Quand le relargage décline, deu standards dopamine ont été injectés. Sur le tracé du bas, une dépolarisation contrôle a été réalisée comme ci-dessus, mais en absence de calcium (plus 0,7 µM EGTA), aucun relargage n'a été mesuré, un standard a été injecté pour vérifier le mélange réactionnel. Dans les deux cas, 10 µl des microfragments prélevés du culot (voir Méthodes) ont été utilisés. Le relargage a été réalisé en solution I contenant de la lactopéroxydase et du luminol, comme décrit dans Méthodes.

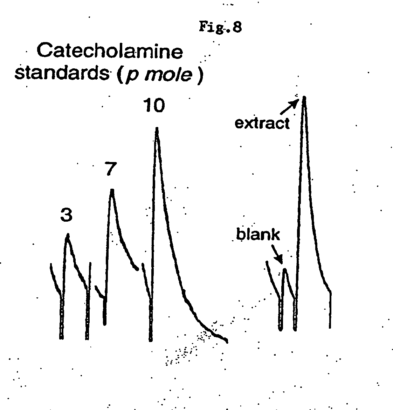

La Figure 8 représente la détermination du contenu en catécholamine des extraits biologiques

L'extrait lavé au TCA et à l'éther a aussi été traité au chloroforme et au Triton, comme décrit dans Méthodes. Le contenu en catécholamine de l'extrait du striatum est comparé aux standards (gauche) et à un blanc traité exactement comme l'échantillon.

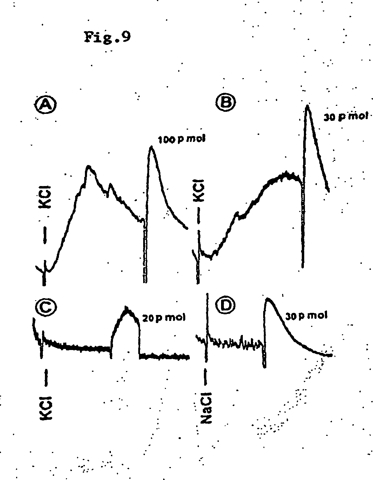

La Figure 9 représente le relargage de catécholamine par des fragments de glande surrénale.

Chaque glande surrénale de souris a été découpée en 4 morceaux lavés dans un grand volume (400 ml) de Krebs mammifère. Les morceaux ont été transférés dans la solution I (A) ou la solution II (B) et le relargage provoqué par la dépolarisation par le XC1 (60 mM) en présence de Calcium (2,5 5 mM). Les proportions de lactopéroxydase et de luminol sont données dans la partie Méthodes. Les tracés du bas sont des contrôles réalisés en absence de Calcium (C) ou en absence de dépolarisation (D) en utilisant du NaCl 60 mM.

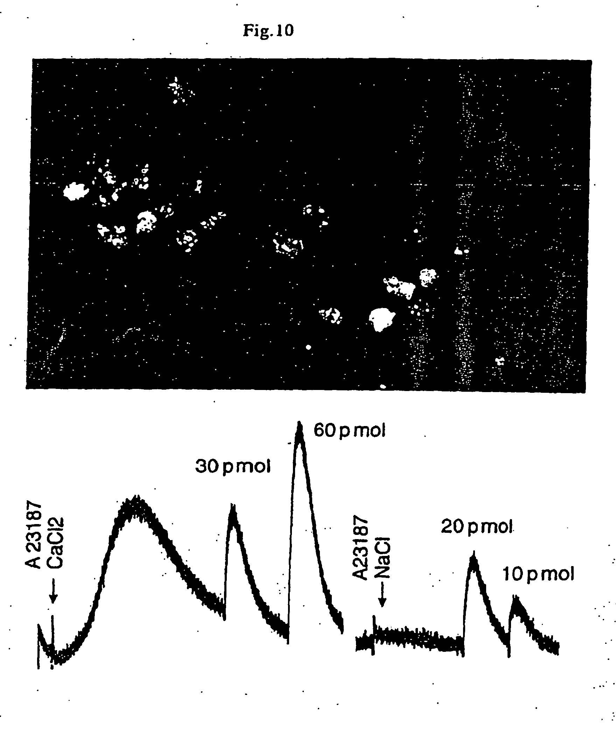

La Figure 10 représente le relargage de catécholamine par des cellules de neuroblastome enrichies en dopamine au cours de leur culture.

Haut : démonstration histochimique de la présence de dopamine dans les cellules NG108-15 enrichies en culture avec de la dopamine (2 mM). Le procédé à l'acide glyoxylique donné un composé fluorescent avec la dopamine. Les cellules ont été enrichies avec 2 à 10 mM de dopamine.

Bas : relargage de dopamine par les cellules enrichies: Les cellules NG108-15 ont été enrichies avec de la dopamine 10 mM ajoutée pendant 12 heures à la culture. Des cellules de quatre récipients à confluence ont été rassemblées, lavées au moins quatre fois dans une solution saline et suspendues dans 0,5 ml de solution saline.

Gauche : le relargage a été mesuré sur 5 µl de suspension cellulaire en solution II contenant de la lactopéroxydase et du luminol comme décrit dans Méthodes. Le relargage a été provoqué par le calcium (1,6 mM) et A23187 (0,7 µM). Deux standards dopamine ont été injectés pour calibrer le relargage.

Droite : contrôle, les cellules enrichies en dopamine n'en ont pas relargué en absence de calcium (l'injection de NaCl remplace le calcium). Le standard montre que le mélange réactionnel aurait détecté toute dopamine relarguée_

### 2) Résultats

### a) Test des catécholamines luminescentes.

Le test décrit résulte d'observations expérimentales montrant que la dopamine ou la norepinéphrine déclenchent l'oxydation chimiluminescente du luminol en présence de lactopéroxydase. L'interprétation la plus simple de la réaction est de considérer que la lactopéroxidase a une activité oxydase formant, à partir des catécholamines et de l'oxygène moléculaire, du péroxyde d'hydtogène qui réagit avec le luminol, la lactopéroxydase catalyse cette réaction chimiluminescente comme le ferait n'importe quelle péroxydase. La Figure 3d schématise la fonction oxydase - péroxydase possible de la lactopéroxyase. La Figure 3e illustre deux courbes dose-réponse obtenues avec la norépinéphrine et la dopamine. La différence entre les deux courbes reflète seulement les propriétés de la préparation d'enzyme et de mélange utilisé dans les deux expériences. Au dessous des courbes sont montrés des éclairs de lumière typiques obtenus dans la gamme de 0,5 à 10 picomoles pour la dopamine. Des réponses équivalentes ont été enregistrées pour la norepinéphrine ou l'épinéphrine. Le test ne mesure pas la L-DOPA qui présente une augmentation lente de la ligne de base. La L-DOPA a inactivé le mélange de réaction, qui a perdu sa sensibilité à l'addition subséquente de norépinéphrine ou de dopamine. La réaction ne détecte pas la sérotonine.
L'évaluation du contenu en catécholamines d'échantillons biologiques inconnus demande une extraction, et le traitement des échantillons qui diminue ou minimise les interférences possibles avec d'autres composés susceptibles d'être présents dans l'extrait, et particulièrement les lipides. L'échantillon lavé au TCA et à l'éther a aussi été mélangé avec du chloroforme, et une faible quantité de Triton a été ajoutée à la phase aqueuse, comme décrit dans Méthodes. La figure 8 montre une évaluation typique, dans laouelle le contenu en catécholamines de l'échantillon est comparé à des standards, un blanc est aussi réalisé avec la solution traitée exactement comme l'extrait biologique. Le tableau I ci-dessous donne les résultats pour cinq expériences différentes, le contenu en catécholamines, trouvé avec le test chimiluminescence est comparé à des valeurs trouvées dans la littérature en utilisant la fluorométrie ou d'autres méthodes.

**Tableau I**

| | Méthode chimiluminescente fluorométrique | Méthode |
|---|---|---|
| Glande surrénale | 490 ± 94 | 1100 * |
| Striatum | 40 ± 10 | 54 ± 8,2 ** |
| Canal déférent | 11,9 ± 0,8 | 7,9 *** |

| | | |
|---|---|---|
| Méthode chimiluminescence : moyenne ± SEM de 5 expériences | | |
| différentes (µg par g de tissu frais). | | |
| * : Udenfriend (1962) | | |
| ** : Cattabeni et al (1972) | | |
| *** : cooper et al. (1978) | | |

### b) Relargage des catécholamines des tissus et des cellules.

Comme décrit précédemment pour les procédés chimiluminescents, le test des catécholamines peut être adapté pour suivre le relargage à partir de coupes de tissus ou de cellules. Ceci a demandé quelques modifications en fonction de chacune des situations expérimentales. Dans le cas de coupes de surrénales, par exemple, l'une des difficultés est venue de la présence de quantités extracellulaires élevées qui épuisent le mélange réactionnel. Dans le cas de suspensions cellulaires, la turbidité de la solution et le matériel lipidique éteignent la chimiluminescence, et diminuent la sensibilité. Les inventeurs ont finalement pu adapter la procédure pour chaque expérience.

### c) Relargaqe des catécholamines par des morceaux de surrénales

Chaque glande a été coupée en 4 ou 5 tranches lavées deux fois dans un grand volume de Krebs mammifères (voir Méthodes). Une tranche a été doucement transférée dans un mélange réactionnel, de la solution **I** ou de la solution II tamponnée à pH 8,6 et contenant de la lactopéroxydase et du luminol à des concentrations plus élevées, comme décrit dans Méthodes. Le relargage a été provoqué par dépolarisation au KCl en présence de calcium. La Figure 9 (A, B, C, D) montre qae le relargage a été obtenu aussi bien dans une solution saline (A) que dans une solution sucrose-Tris (B), et qu'il est clairement dépendant du calcium. Des contrôles réalisés en absence de calcium ou après avoir injecté du NaCl au lieu de KCl, sont montrés au bas de la Figure 9 (C et D).

### d) Relargage des catécholamines par des microfragments striataux

La procédure a aussi été testée sur des tranches striatales, prélevées sur des cerveaux de souris. Dans ce cas également, les fragments ont été lavés dans un grand volume de Krebs et laissés sédimenter avant de les collecter dans 2 ml de Krebs. Une aliquote de matériel est prélevée du culot et injectée dans lé mélange réactionnel. Dans ce cas, les proportions de luminol et de lactopéroxydase utilisés dans le test (voir Méthodes) qui donnent le maximum de sensibilité ont été gardées. Le relargage a également été provoqué par dépolarisation au KCl (Figure 7, tracé du haut), un contrôle en absence de calcium n'a pas montré de relargage de dopamine (Figure 7, tracé du bas). Dans les deux cas, un standard à la dopamine a montré que le mélange réactionnel était sensible à la dopamine et qu'il permet une évaluation du relargage Ca Dépendant du transmetteur.

### Relargage des catécholamines de suspensions cellulaires

Après avoir incubé les morceaux de surrénales dans du Krebs puis les avoir lavés pour enlever les substances interférentes, les morceaux ont été dissociés par plusieurs aspirations à la pipette ; la suspension obtenue à partir de 4 glandes surrénales a été recueillie dans un volume de 2 ml de Krebs. Le relargage a été réalisé en utilisant 5 à 10 µl de solution, l'utilisation de davantage de cellules éteint le mélange réactionnel. La Figure 6 A et B a pour but de comparer le relargage de cellules de surrénales dans deux solutions différentes, solution I saline et solution II non saline. En A, le relargage à partir des cellules a été mesuré en solution saline et provoqué par le calcium ionophore (A23187) en présence de calcium 2,5 mM. Quand le calcium est réduit à 10 µM, le relargage décline mais n'est pas négligeable (tracé du milieu). Le tracé le plus bas montre la perte spontanée en absence d'ionophore ou de calcium.

La Figure 6 B montre qu'en milieu non salin (solution II : sucrose Tris), le relargage de ces cellules peut encore être provoqué comme en A par l'ionophore A23187 et le calcium, mais la dépendance au calcium est plus évidente puisque le relargage est devenu négligeable en conditions de faible niveau de calcium. De plus, les pertes spontanées n'ont pas été détectées en sucrose-Tris. Il est probable qu'un comportement de relargage non dépendant du calcium, dû au gonflement des cellules, a lieu dans la solution saline.

### f) Relargage de dopamine de cellules en culture enrichies en dopamine

Des travaux antérieurs ont montré que les cellules en culture pouvaient être enrichies en ACh, Glu ou Gaba en ajoutant le transmetteur dans le milieu de culture (Israel et al., 1994, 1997). Dans le cas particulier de cellules NG108-15, les inventeurs ont trouvé des articles montrant qu'elles pouvaient incorporer de la sérotonine ou des catécholamines (Furuya et al., 1985, Liu et al., 1992). Ils montrent ici que les cellules NG108-15 accumulent la dopamine du milieu de culture auquel 2 à 10 mM de dopamine ont été ajoutés. Les catécholamines doivent être protégées de l'auto oxydation en utilisant de l'acide ascorbique, en particulier pour les longues périodes d'incubation (voir Méthodes). La Figure 10 (haut) montre que les cellules sont réellement enrichies en dopamine. La caractérisation histochimique de la dopamine avec la procédure à l'acide glyoxylique rend les cellules fluorescentes. Les cellules chargées ont été capables de relarguer le neurotransmetteur en réponse à l'influx de calcium provoqué par A23187 en présence de calcium. Dans ce cas, il était nécessaire de renforcer le mélange réactionnel après avoir ajouté les cellules dans la solution II contenant les réactifs et l'ionophore. La dopamine extracellulaire est d'abord mesurée, et quand la ligne de base est atteinte, une quantité supplémentaire de luminol, de lactopéroxydase et 1 ml de solution II (voir Méthodes) a été ajouté. Quand la ligne de base a été atteinte, le relargage a été provoqué avec du calcium ((Figure 10, tracé de gauche). Un contrôle dans lequel une injection de NaCl est réalisée à la place du calcium est montré en Figure 10 (tracé de droite). Un relargage de dopamine clairement calcium-dépendant a été obtenu des cellules chargées.

### 3) Discussion.

Quand le présent projet a débuté, les inventeurs souhaitaient coupler l'oxydation des catécholamines par la monoamine oxydase à la détection de péroxyde d'hydrogène à l'aide du luminol. Il a été trouvé par hasard que la lactopéroxydase était en elle-même suffisante pour catalyser à la fois l'oxydation des catécholamines et la détection du produit avec la chimiluminescence du luminol. Il est connu que les catécholamines sont détectées par des procédures ampérométriques, et que ces méthodes sont sensibles, la spécificité dépend de la relation entre le potentiel d'oxydoréduction des catécholamines et l'électrode de détection (Albillos et al., 1977, Pan et al., 1977). Dans le présent test, il est probable que l'oxydoréduction a lieu par l'intermédiaire du fer de la lactopéroxydase, il pourrait servir comme donneur d'électrons dans l'étape « Oxydase » et de d'accepteur d'électron dans l'action de la péroxydase sur le luminol. Dans tous les cas, l'affinité de FeCl3 pour les catécholamines est à la base de la « réaction de vulpian « qui a permis de découvrir l'adrénaline dans la circulation adrénergique. Il est alors probable que l'ion métallique de la péroxydase est essentiel pour expliquer le mécanisme des réactions observées.

Les inventeurs ont aussi observé que la procédure ne distingue pas la norepinéphrine, l'épinéphrine ou la dopamine, et que L-DOPA n'est pas mesurable, en dépit du fait qu'elle inactive le mélange réactionnel. D'autres transmetteurs, tels que la sérotonine oxydée par la monoamine oxydase, n'ont pas réagi lors du test à la lactopéroxydase. D'autres composés, l'ascorbate ou l'acétylcholine, ont peu ou pas d'effet.

Le procédé décrit peut être utilisé en camplément d'autres méthodes. Le procédé est rapide et complète les autres méthodes développées pour l'ATP, l'ACh, le glutamate et le Gaba en utilisant le même luminomètre pour déterminer l'émission de lumière.

Les inventeurs ont aussi été capables de contrôler le relargage de catécholamines de diverses préparations adrénergiques. Le procédé permet de détecter en continu le relargage. L'un des résultats pouvant être intéressant dans le futur est l'observation que certaines cellules peuvent être enrichies en dopamine lors de la culture. Ceci pourrait permettre de trouver les cellules capables d'accumuler et de relarguer ce transmetteur, dans le but de corriger, un jour, un déficit dopaminergique comme dans la maladie de Parkinson (Bjorklund et Stenevi, 1985 ; Espejo et al., 1998). De plus, la méthode pourrait être utile pour trouver des composés pharmacologiques qui affectent la transmission adrénergique.

### REFERENCES BIBLIOGRAPHIQUES

Albillos A, Dernick G, Horstmann H, Almers W, Alvarez de Toledo G, Lindau M. The exocytotic event in chromaffin cells revealed by patch amperometry. Nature 1997;389:509-512.

Bjorklund A, Steveni U. Intracerebral neuronal grafting. In : Neural grafting in the mammalian CNS, A. Bjorklund and U. Steveni Eds. Amsterdam Elsevier. 1985;3-14.

Cadet J, Brannock C. Free radicals and the pathobiology of brain dopamine systems. Neurochem Int 1998,32:117-131.

Cattabeni F, Koslow SH, Costa E. Studies of neurotransmitters at. the synaptic level. Edited by Costa E. Iversen L.L. and Paoletti R. In : Advances in biochemical psychopharmacology. Raven press NY. 1972;6:51

Cooper JR, Bloom FE, Roth RH. In : Biochemical basis of neuropharmacology (third edition). Oxford University Press 1978;117.

De Luca M, McElroy WD. Purification and properties of firefly luciferase. In Methods in enzymology bioluminescence and chemiluminescence. Ed M.A. De Luca Acad Press NY. 1978;LVII:3-15.

De La Torre JC. An imporved approach to histofluorescence using the SPG method for tissue monoamines. J Neurosci Method 1980;3:1-5.

Espejo EF, Montoro RJ, Armengol AJ, Lopez-Barneo J. Cellular and functional recovery of Parkinsonian Rats after transplantation of carotid body cell aggregates. Neuron 1998;20:197-206.

Fosse MV, Kolstad J, Fonnum F. A bioluminescence method for the measurement of L-glutamate: application to the study of changes in the release of L-glutamate from lateral geniculate nucleus and superior colliculus after visual cortex ablation in rats. Journal of Neurochemistry. 1986;47;340-349.

Furuya S, Sawada M, Nagatsu T, Suzuki O, Higashida H. Localization of [³H] serotonin in neuroblastoma x Glioma hybrid cells. Brain Research 1985;361:77-90.

Israël M, Lesbats B. Continuous determination by a chemiluminescent method of acetylcholine release and compartmentation in *Torpedo* electric organ synaptosomes. Journal of Neurochemistry 1981 a;37:1475-1483.

Israël M, Lesbats B. Chemiluminescent determination of acetylcholine and continuous detection of it release from *Torpedo* electric organ synapses and synaptosomes. Neurochem Int. 1981 b;3:81-90.

Israël M, Lesbats B, Application to mammalian tissues of the chemiluminescent method for detecting acetylcholine. Journal of Neurochemistry. 1982;39:248-250.

Israël M, Lesbats B. The use of bioluminescence techniques in neurobiology, with emphasis on the cholinergic system. In : Neurochemistry, a practical approach (Turner AJ Bachelard HS Eds) IRL Press, Oxford 1987;113-135.

Israël M, Lesbats B. A bioluminescent aminobutyrate assay for monitoring its release from inhibitory nerve endings. Journal of Neurochemistry 1996;67:2624-2627.

Israël M, Lesbats B, Bruner J. Glutamate and acetylcholine release from cholinergic nerve terminals, a calcium control of the specificity of the release mechanism. Neurochem Int 1993;22:53-58.

Israël M, Lesbats B, Synguelakis M, Joliot A. Acetylcholine accumulation and release by hybrid NG108-15, glioma and neuroblastoma cells - role of a 15 kDa membrane protein in release. Neurochem Int 1994,25;103-109.

Israël M, Lesbats B, Tomasi M, Couraud PO, Vignais L, Quinonero J, Tchelingerian JL. Calcium-dependent release specificities of various cell lines loaded with different transmistters. Neuropharmacology 1997;36:1789-1793.

Knigge KM, Hoffman G, Scott DE, Sladek JR. Identification of catecholamine and luteinizing hormone-releasing hormone (LHRH) containing neurons in primary cultures of dispersed cells of the basal hypothalamus. Brain Res 1977;120:393-405.

Liu Y, Peter D, Roghani A, Schuldiner S, Prive GG, Eisenberg D, Brecha N, Edwards RH. A cDNA that suppresses MMP⁺ toxicity encodes a vesicular amine transporter. Cell 1992;70:539-551.

Michelson AM. Purification and properties of Pholas dactylus Luciferine and Luciferase. In Methods in enzymology bioluminescence and chemiluminescence Ed. M. A. De Luca; Acad Press NY 1978;LVII:385-406.

Meunier FM, Israël M, Lesbats B. Release of ATP from stimulated nerve electroplaque junction. Nature 1975;257:407-408.

Pan CY, Kao LS. Catecholamine secretion from bovine Adrenal Chomaffin cells : The role of Na⁺/Ca²⁺ Exchange and the intracellular Ca²⁺pool. J. Neurochem 1997;69:1085-1092.

Takayama M, Itoh S, Nagasaki T and Tanimizu I. A new enzymatic method for détermination of serum Choline-containing phospholipids. Clinica Chimica Acta 1977;79:93-98.

Tanne M, Finberg JP, Youdim MB, Ulitzur S. A New rapid ans sensitive bioliminescence assay for monoamine oxidase activity. J. Neurochem 1985;44:1378-1384.

Tenne M, Youdim MB. Ulitzur S, Finberg JP. Deamination of aliphatic amines by monoamine oxidase A and B studies using a bioluminescence technique. J Neurochem 1985;44:1373-1377.

Udenfriend S. In fluorescence assay in biology and medecine. Acad. Press NY 1962;1:158.

## Revendications

1. Méthode pour la préparation de microcubes calibrés de matière cérébrale de mammifère comprenant les étapes suivantes :
(i) sélectionner un cerveau entier de mammifère,
(ii) le découper en cubes,
(iii) laver les cubes dans un volume de milieu de Krebs mammifères,
(iv) passer les cubes à travers une ou plusieurs grilles dont les mailles ont un diamètre défini,
(v) récupérer les microcubes calibrés.

2. Méthode selon la revendication 1, **caractérisée en ce que** ledit mammifère est sélectionné parmi le rat, la souris et l'homme.

3. Méthode selon l'une quelconque des revendications 1 à 2, **caractérisée en ce que** ladite grille est une grille en nylon ou métallique.

4. Méthode selon l'une quelconque des revendications 1 à 3 **caractérisée en ce que** ledit diamètre des grilles est compris entre 0,01 et 2 mm².

5. Méthode selon la revendication 4 **caractérisée en ce que** ledit diamètre des gilles est 1 mm².

6. Composition comprenant des microcubes calibrés de matière cérébrale de mammifère **caractérisée en ce que** lesdits microcubes calibrés sont préparés selon la méthode de l'une quelconque des revendications 1 à 5.

7. Composition selon la revendication 6, **caractérisée en ce que** lesdits microcubes ont une taille moyenne comprise entre 0,1 et 5 mm³.

8. Composition selon la revendication 6, **caractérisée en ce que** lesdits microcubes ont une taille moyenne comprise entre 0,1 et 3 mm³.

9. Composition selon la revendication 6, **caractérisée en ce que** lesdits microcubes ont une taille moyenne de 1 mm³ environ.

10. Composition selon l'une quelconque des revendications à 6 à 9, **caractérisée en ce que** lesdits microcubes sont dans un milieu de Krebs.

11. Procédé d'identification de composés capables de moduler la libération d'au moins un neuromédiateur, ledit procédé comprenant les étapes suivantes :
(i) placer en contact au moins un composé à tester avec une composition de microcubes calibrés de matière cérébrale de mammifère selon l'une quelconque des revendications 6 à 10,
(ii) déterminer si le composé à tester module la libération dudit neuromédiateur par ladite préparation en comparaison à un essai réalisé dans les mêmes conditions en absence de composé à tester.

12. Procédé selon la revendication 11, **caractérisé en ce que** l'étape (ii) consiste à mettre en contact la composition de l'étape (i), ou son surnageant, avec une ou plusieurs substances dont l'une au moins est capable de réagir avec un neuromédiateur libéré, et à doser ou détecter au moins un produit résultant de ladite réaction.

13. Procédé selon la revendication 12, **caractérisé en ce que** ledit produit est une substance détectable par mesure de lumière émise.

14. Procédé selon la revendication 12, **caractérisé en ce que** l'étape (ii) consiste à mettre en contact la composition de l'étape (i), ou son surnageant, avec une ou plusieurs enzyme dont le substrat de l'une au moins est un desdits neuromédiateurs, et avec au moins un agent capable d'émettre de la lumière suite à la dégradation dudit neuromédiateur par son enzyme.

15. Procédé selon l'une quelconque des revendications 11 à 14, **caractérisé en ce que** ledit composé à tester est sélectionné parmi un composé isolé, un mélange de composés, un échantillon biologique, une banque combinatoire, une molécule synthétique ou naturelle.

16. Procédé selon l'une quelconque des revendications 11 à 15, **caractérisé en ce que** ledit neuromédiateur est choisi parmi le glutamate (Glu), l'acétylcholine (Ach), le γ- aminobutyrate (Gaba) et l'ATP.

17. Procédé selon l'une quelconque des revendications 11 à 16, **caractérisé en ce que** ledit neuromédiateur est l'acétylcholine et **en ce que** l'étape (ii) consiste à mettre en contact la composition de l'étape (i), ou son sumageant, successivement ou simultanément, avec une acétylcholinestérase, une cholineoxydase, une peroxydase, et du luminol, et à détecter ou mesurer la lumière émise.

18. Procédé selon l'une quelconque des revendications 11 à 16, **caractérisé en ce que** ledit neuromédiateur est le glutamate et **en ce que** l'étape (ii) consiste à mettre en contact la composition de l'étape (i), ou son surnageant, successivement ou simultanément, avec une glutamate déshydrogénase, une oxydoréductase, une luciférase, du NAD et un décaldéhyde, et à détecter ou mesurer la lumière émise.

19. Procédé selon l'une quelconque des revendications 11 à 16, **caractérisé en ce que** ledit neuromédiateur est le γ-aminobutyrate et **en ce que** l'étape (ii) consiste à mettre en contact la composition de l'étape (i), ou son surnageant, successivement ou simultanément, avec une gabase, une oxydoréductase, une luciférase, du NAD et de FMN, et à détecter ou mesurer la lumière émise.

20. Procédé selon l'une quelconque des revendications 11 à 16, **caractérisé en ce que** ledit neuromédiateur est l'ATP et **en ce que** l'étape (ii) consiste à mettre en contact la composition de l'étape (i), ou son surnageant, avec une luciférase, et à détecter ou mesurer la lumière émise.

## Patentansprüche

1. Verfahren zur Herstellung von kalibrierten Mikrowürfeln aus Säugetier-Hirnmaterial, welches die folgenden Schritte umfasst:
(i) ein vollständiges Säugetier-Gehirn auszuwählen;
(ii) dieses in Würfel zu schneiden
(iii) die Würfel in einem Volumen Krebs-Säugetier-Medium zu waschen,
(iv) die Würfel durch ein oder mehrere Gitter, deren Maschen einen definierten Durchmesser haben, hindurchzuleiten,
(v) die kalibrierten Mikrowürfel zu gewinnen.

2. Verfahren nach Anspruch 1, **dadurch gekennzeichnet, dass** das Säugetier unter der Ratte, der Maus und dem Menschen ausgewählt wird.

3. Verfahren nach einem der Ansprüche 1 bis 2, **dadurch gekennzeichnet, dass** das Gitter ein Nylon- oder Metallgitter ist.

4. Verfahren nach einem der Ansprüche 1 bis 3, **dadurch gekennzeichnet, dass** der Durchmesser der Gitter zwischen 0,01 und 2 mm² eingeschlossen beträgt.

5. Verfahren nach Anspruch 4, **dadurch gekennzeichnet, dass** der Durchmesser der Gitter 1 mm² beträgt.

6. Zusammensetzung, umfassend kalibrierte Mikrowürtel aus Säugetier-Hinmaterial, **dadurch gekennzeichnet, dass** die kalibrierten Mikrowürfel gemäß dem Verfahren nach einem der Ansprüche 1 bis 5 hergestellt werden.

7. Zusammensetzung nach Anspruch 6, **dadurch gekennzeichnet, dass** die Mikrowürfel eine mittlere Größe zwischen 0,1 und 5 mm³ eingeschlossen haben.

8. Zusammensetzung nach Anspruch 6, **dadurch gekennzeichnet, dass** die Mikrowürfel eine mittlere Größe zwischen 0,1 und 3 mm³ eingeschlossen haben.

9. Zusammensetzung nach Anspruch 6, **dadurch gekennzeichnet, dass** die Mikrowürfel eine mittlere Größe von ungefähr 1 mm³ haben.

10. Zusammensetzung nach einem der Ansprüche 6 bis 9, **dadurch gekennzeichnet, dass** die Mikrowürfel sich in einem Krebs-Medium befinden.

11. Verfahren zur Identifizierung von Verbindungen, die in der Lage sind, die Freisetzung von wenigstens einem Neuromediator zu modulieren, wobei das Verfahren die folgenden Schritte umfasst:
(i) wenigstens eine zu testende Verbindung mit einer Zusammensetzung von kalibrierten Mikrowürfeln aus Säugetier-Himmaterial nach einem der Ansprüche 6 bis 10 in Kontakt zu bringen,
(ii) zu bestimmen, ob die zu testende Verbindung die Freisetzung des Neuromediators durch die Zubereitung im Vergleich zu einem unter den gleichen Bedingungen in Abwesenheit von zu testender Verbindung ausgeführten Versuch moduliert.

12. Verfahren nach Anspruch 11, **dadurch gekennzeichnet, dass** der Schritt (ii) darin besteht, die Zusammensetzung des Schritts (i) oder deren Überstand mit einer oder mehreren Substanzen, von denen wenigstens eine in der Lage ist, mit einem freigesetzten Neuromediator zu reagieren, in Kontakt zu bringen und wenigstens ein Produkt, welches aus der Reaktion resultiert, quantitativ zu bestimmen oder nachzuweisen.

13. Verfahren nach Anspruch 12, **dadurch gekennzeichnet, dass** das Produkt eine durch Messung von emittiertem Licht nachweisbare Substanz ist.

14. Verfahren nach Anspruch 12, **dadurch gekennzeichnet, dass** der Schritt (ii) darin besteht, die Zusammensetzung des Schritts (i) oder deren Überstand mit einem oder mehreren Enzymen, wobei das Substrat von diesem wenigstens einen einer der genannten Neuromediatoren ist, und mit wenigstens einem Mittel, welches in der Lage ist, im Anschluss an den Abbau des Neuromediators durch sein Enzym Licht zu emittieren, in Kontakt zu bringen.

15. Verfahren nach einem der Ansprüche 11 bis 14, **dadurch gekennzeichnet, dass** die zu testende Verbindung unter einer isolierten Verbindung, einer Mischung von Verbindungen, einer biologischen Probe, einer kombinatorischen Bibliothek, einem synthetischen oder natürlichen Molekül ausgewählt wird.

16. Verfahren nach einem der Ansprüche 11 bis 15, **dadurch gekennzeichnet, dass** der Neuromediator unter Glutamat (Glu), Acetylcholin (Ach), γ-Aminobutyrat (Gaba) und ATP ausgewählt wird.

17. Verfahren nach einem der Ansprüche 11 bis 16, **dadurch gekennzeichnet, dass** der Neuromediator Acetylcholin ist und dass der Schritt (ii) darin besteht, die Zusammensetzung des Schritts (i) oder deren Überstand nacheinander oder gleichzeitig mit einer Acetylcholinesterase, einer Cholinoxidase, einer Peroxidase und Luminol in Kontakt zu bringen und das emittierte Licht nachzuweisen oder zu messen.

18. Verfahren nach einem der Ansprüche 11 bis 16, **dadurch gekennzeichnet, dass** der Neuromediator Glutamat ist und dass der Schritt (ii) darin besteht, die Zusammensetzung des Schritts (i) oder deren Überstand nacheinander oder gleichzeitig mit einer Glutamatdehydrogenase, einer Oxidoreduktase, einer Luciferase, NAD und einem Decaldehyd (Decanal) in Kontakt zu bringen und das emittierte Licht nachzuweisen oder zu messen.

19. Verfahren nach einem der Ansprüche 11 bis 16, **dadurch gekennzeichnet, dass** der Neuromediator γ-Aminobutyrat ist und dass der Schritt (ii) darin besteht, die Zusammensetzung des Schritts (i) oder deren Überstand nacheinander oder gleichzeitig mit einer Gabase, einer Oxidoreduktase, einer Luciferase, NAD und FMN in Kontakt zu bringen und das emittierte Licht nachzuweisen oder zu messen.

20. Verfahren nach einem der Ansprüche 11 bis 16, **dadurch gekennzeichnet, dass** der Neuromediator ATP ist und dass der Schritt (ii) darin besteht, die Zusammensetzung des Schritts (i) oder deren Überstand mit einer Luciferase in Kontakt zu bringen und das emittierte Licht nachzuweisen oder zu messen.

## Claims

1. Method for preparing calibrated microcubes of mammalian brain material, comprising the following steps:
(i) selecting a whole mammalian brain,
(ii) cutting it into cubes,
(iii) washing the cubes in a volume of mammalian Krebs medium,
(iv) passing the cubes through one or more screens, the mesh sizes of which have a defined diameter,
(v) recovering the calibrated microcubes.

2. Method according to Claim 1, **characterized in that** said mammal is selected from rats, mice and humans.

3. Method according to either one of Claims 1 and 2, **characterized in that** said screen is a nylon or metal screen.

4. Method according to any one of Claims 1 to 3, **characterized in that** said diameter of the screens is between 0.01 and 2 mm².

5. Method according to Claim 4, **characterized in that** said diameter of the screens is 1 mm².

6. Composition comprising calibrated microcubes of mammalian brain material, **characterized in that** said calibrated microcubes are prepared according to the method of any one of Claims 1 to 5.

7. Composition according to Claim 6, **characterized in that** said microcubes have a mean size of between 0.1 and 5 mm³.

8. Composition according to Claim 6, **characterized in that** said microcubes have a mean size of between 0.1 and 3 mm³.

9. Composition according to Claim 6, **characterized in that** said microcubes have a mean size of approximately 1 mm³.

10. Composition according to any one of Claims 6 to 9, **characterized in that** said microcubes are in a Krebs medium.

11. Process for identifying compounds capable of modulating the release of at least one neuromediator, said process comprising the following steps:
(i) placing at least one compound to be tested in contact with a composition of calibrated microcubes of mammalian brain material according to any one of Claims 6 to 10,
(ii) determining whether the compound to be tested modulates the release of said neuromediator by said preparation, compared with a trial carried out under the same conditions in the absence of compound to be tested.

12. Process according to Claim 11, **characterized in that** step (ii) consists in bringing the composition of step (i), or its supernatant, into contact with one or more substances, at least one of which is capable of reacting with a released neuromediator, and in assaying or detecting at least one product resulting from said reaction.

13. Process according to Claim 12, **characterized in that** said product is a substance that can be detected by measuring emitted light.

14. Process according to Claim 12, **characterized in that** step (ii) consists in bringing the composition of step (i), or its supernatant, into contact with one or more enzymes, the substrate of at least one of which is one of said neuromediators, and with at least one agent capable of emitting light subsequent to the degradation of said neuromediator by its enzyme.

15. Process according to any one of Claims 11 to 14, **characterized in that** said compound to be tested is selected from an isolated compound, a mixture of compounds, a biological sample, a combinatorial library, a synthetic molecule or a natural molecule.

16. Process according to any one of Claims 11 to 15, **characterized in that** said neuromediator is chosen from glutamate (Glu), acetylcholine (Ach), γ-aminobutyrate (Gaba) and ATP.

17. Process according to any one of Claims 11 to 16, **characterized in that** said neuromediator is acetylcholine and **in that** step (ii) consists in bringing the composition of step (i), or its supernatant, into contact, successively or simultaneously, with an acetylcholinesterase, a choline oxydase, a peroxydase and luminol, and in detecting or measuring the emitted light.

18. Process according to any one of Claims 11 to 16, **characterized in that** said neuromediator is glutamate and **in that** step (ii) consists in bringing the composition of step (i), or its supernatant, into contact, successively or simultaneously, with a glutamate dehydrogenase, an oxydoreductase, a luciferase, NAD and a decaldehide, and in detecting or measuring the emitted light.

19. Process according to any one of Claims 11 to 16, **characterized in that** said neuromediator is γ-aminobutyrate and **in that** step (ii) consists in bringing the composition of step (i), or its supernatant, into contact, successively or simultaneously, with a gabase, an oxydoreductase, a luciferase, NAD and FMN, and in detecting or measuring the emitted light.

20. Process according to any one of Claims 11 to 16, **characterized in that** said neuromediator is ATP and **in that** step (ii) consists in bringing the composition of step (i), or its supernatant, into contact with a luciferase, and in detecting or measuring the emitted light.
